# EUROPEAN PATENT SPECIFICATION

(11) **EP 1 637 598 B1**
(45) Date of publication and mention of the grant of the patent: **05.07.2017**
(21) Application number: 04735526.8
(22) Date of filing: 31.05.2004
(51) Int. Cl.: C12N 15/09, C12P 21/08, A61K 39/395, A61P 9/10, A61P 35/04

(54) **CD47 PARTAL PEPTIDE AND ANTI-SHPS-1 MONOCLONAL ANTIBODY**
CD47-TEILPEPTID UND MONOKLONALER ANTI-SHPS-1-ANTIKÖRPER
PEPTIDE PARTIEL DE CD47 ET ANTICORPS MONOCLONAL ANTI-SHPS-1

(30) Priority: 02.06.2003 JP 2003157287
(43) Date of publication of application: 22.03.2006
(73) Proprietor: National University Corporation Gunma University, Maebashi-shi Gunma (JP)
(72) Inventor: MATOZAKI, Takashi, Maebashi-shi, Gunma 371-0047 (JP); OKAZAWA, Hideki, Graceful Mansion 305, Maebashi-shi, Gunma 371-0031 (JP)
(74) Representative: Owen, Deborah Jane
(86) International application number: PCT/JP2004/007871
(87) International publication number: WO 2004/108923

(56) References cited:
- WO-A-01/48020
- WO-A-02/092784
- WO-A1-97/27873
- JP-A- 11 504 819
- JP-A- 2001 512 417
- VERNON-WILSON E F ET AL: "CD47 IS A LIGAND FOR RAT MACROPHAGE MEMBRANE SIGNAL REGULATORY PROTEIN SIRP (OX41) AND HUMAN SIRPALPHA1" EUROPEAN JOURNAL OF IMMUNOLOGY, WEINHEIM, vol. 30, 1 August 2000 (2000-08-01), pages 2130-2137, XP000984773 ISSN: 0014-2980
- SEIFFERT M ET AL: "HUMAN SIGNAL-REGULATORY PROTEIN IS EXPRESSED ON NORMAL, BUT NOT ON SUBSETS OF LEUKEMIC MYELOID CELLS AND MEDIATES CELLULAR ADHESION INVOLVING ITS COUNTERRECEPTOR CD47" BLOOD, W.B.SAUNDERS COMPANY, ORLANDO, FL, vol. 94, no. 11, 1 December 1999 (1999-12-01), pages 3633-3643, XP000973562 ISSN: 0006-4971
- OSHIMA KUMI ET AL: "SHPS-1: a budding molecule against cancer dissemination." CANCER RESEARCH 15 JUL 2002, vol. 62, no. 14, 15 July 2002 (2002-07-15), pages 3929-3933, XP002481061 ISSN: 0008-5472
- MAILE LAURA A ET AL: "The association between integrin-associated protein and SHPS-1 regulates insulin-like growth factor-I receptor signaling in vascular smooth muscle cells" MOLECULAR BIOLOGY OF THE CELL, BETHESDA, MD, US, vol. 14, no. 9, 1 September 2003 (2003-09-01), pages 3519-3528, XP002423400 ISSN: 1059-1524
- MOTEGI SEI-ICHIRO ET AL: "Role of the CD47-SHPS-1 system in regulation of cell migration." EMBO (EUROPEAN MOLECULAR BIOLOGY ORGANIZATION) JOURNAL, vol. 22, no. 11, 2 June 2003 (2003-06-02), pages 2634-2644, XP002481062 ISSN: 0261-4189
- OGURA TAKESHI ET AL: "Resistance of B16 melanoma cells to CD47-induced negative regulation of motility as a result of aberrant N-glycosylation of SHPS-1" JOURNAL OF BIOLOGICAL CHEMISTRY, AL, vol. 279, no. 14, 2 April 2004 (2004-04-02), pages 13711-13720, XP002457666 ISSN: 0021-9258
- FUJIOKA Y, ET AL: 'A novel membrane glycoprotein SHPS-1, that binds the SH2-domain-containing protein tyrosine phosphatase SHP-2 in response to mitogens and cell adhesion' MOLL. CELL. BIOL. vol. 16, no. 12, December 1996, pages 6887 - 6899, XP002072087
- YAMAO T, ET AL: 'Mouse and human SHPS-1: molecular cloning of cDNAs and chromosomal localization of genes' BIOCHEMICAL AND BIOPHYSICAL RESEARCH COMMUNICATIONS vol. 231, 1997, pages 61 - 67, XP000648269
- YAMAO T, ET AL: 'Negative regulation of platelet clearance and of the macrophage phagocytic response by the transmembrane glycoprotein SHPS-1' J. BIOL. CHEM vol. 277, 2002, pages 39833 - 39839, XP002980543

### SEQUENCE LISTING

<110> Japan Science And Technology Agency
<120> CD47 partial peptide (1-142 amino acid)
<130> 04F029PCT
<150> JP2003-157287
   <151> 2003-06-02
<160> 4
<170> PatentIn version 3.1
<210> 1
   <211> 426
   <212> DNA
   <213> Homo sapience
<220>
   <221> CDS
   <222> (1).. (426)
   <223>
<400> 1
   atg tgg ccc ctg gta gcg gcg ctg ttg ctg ggc tcg gcg tgc tgc gga 48
   Met Trp Pro Leu Val Ala Ala Leu Leu Leu Gly Ser Ala Cys Cys Gly
   1 5 10 15
   tca gct cag cta cta ttt aat aaa aca aaa tct gta gaa ttc acg ttt 96
   Ser Ala Gln Leu Leu Phe Asn Lys Thr Lys Ser Val Glu Phe Thr Phe
   20 25 30
   tgt aat gac act gtc gtc att cca tgc ttt gtt act aat atg gag gca 144
   Cys Asn Asp Thr Val Val Ile Pro Cys Phe Val Thr Asn Met Glu Ala
   35 40 45
   caa aac act act gaa gta tac gta aag tgg aaa ttt aaa gga aga gat 192
   Gln Asn Thr Thr Glu Val Tyr Val Lys Trp Lys Phe Lys Gly Arg Asp
   50 55 60
   att tac acc ttt gat gga gct cta aac aag tcc act gtc ccc act gac 240
   Ile Tyr Thr Phe Asp Gly Ala Leu Asn Lys Ser Thr Val Pro Thr Asp
   65 70 75 80
   ttt agt agt gca aaa att gaa gtc tca caa tta cta aaa gga gat gcc 288
   Phe Ser Ser Ala Lys Ile Glu Val Ser Gln Leu Leu Lys Gly Asp Ala
   85 90 95
   tct ttg aag atg gat aag agt gat gct gtc tca cac aca gga aac tac 336
   Ser Leu Lys Met Asp Lys Ser Asp Ala Val Ser His Thr Gly Asn Tyr
   100 105 110
   act tgt gaa gta aca gaa tta acc aga gaa ggt gaa acg atc atc gag 384
   Thr Cys Glu Val Thr Glu Leu Thr Arg Glu Gly Glu Thr Ile Ile Glu
   115 120 125
   cta aaa tat cgt gtt gtt tca tgg ttt tct cca aat gaa aat 426
   Leu Lys Tyr Arg Val Val Ser Trp Phe Ser Pro Asn Glu Asn
   130 135 140
<210> 2
   <211> 142
   <212> PRT
   <213> Homo sapience
<400> 2
   Met Trp Pro Leu Val Ala Ala Leu Leu Leu Gly Ser Ala Cys Cys Gly
   1 5 10 15
   Ser Ala Gln Leu Leu Phe Asn Lys Thr Lys Ser Val Glu Phe Thr Phe
   20 25 30
   Cys Asn Asp Thr Val Val Ile Pro Cys Phe Val Thr Asn Met Glu Ala
   35 40 45
   Gln Asn Thr Thr Glu Val Tyr Val Lys Trp Lys Phe Lys Gly Arg Asp
   50 55 60
   Ile Tyr Thr Phe Asp Gly Ala Leu Asn Lys Ser Thr Val Pro Thr Asp
   65 70 75 80
   Phe Ser Ser Ala Lys Ile Glu Val Ser Gln Leu Leu Lys Gly Asp Ala
   85 90 95
   Ser Leu Lys Met Asp Lys Ser Asp Ala Val Ser His Thr Gly Asn Tyr
   100 105 110
   Thr Cys Glu Val Thr Glu Leu Thr Arg Glu Gly Glu Thr Ile Ile Glu
   115 120 125
   Leu Lys Tyr Arg Val Val Ser Trp Phe Ser Pro Asn Glu Asn
   130 135 140
<210> 3
   <211> 1113
   <212> DNA
   <213> Artificial
<220>
   <223> Fusion of CD47 part(1-142 amino acid) and Ig-Fc
<220>
   <221> CDS
   <222> (1).. (1113)
   <223>
<400> 3
   atg tgg ccc ctg gta gcg gcg ctg ttg ctg ggc tcg gcg tgc tgc gga 48
   Met Trp Pro Leu Val Ala Ala Leu Leu Leu Gly Ser Ala Cys Cys Gly
   1 5 10 15
   tca gct cag cta cta ttt aat aaa aca aaa tct gta gaa ttc acg ttt 96
   Ser Ala Gln Leu Leu Phe Asn Lys Thr Lys Ser Val Glu Phe Thr Phe
   20 25 30
   tgt aat gac act gtc gtc att cca tgc ttt gtt act aat atg gag gca 144
   Cys Asn Asp Thr Val Val Ile Pro Cys Phe Val Thr Asn Met Glu Ala
   35 40 45
   caa aac act act gaa gta tac gta aag tgg aaa ttt aaa gga aga gat 192
   Gln Asn Thr Thr Glu Val Tyr Val Lys Trp Lys Phe Lys Gly Arg Asp
   50 55 60
   att tac acc ttt gat gga gct cta aac aag tcc act gtc ccc act gac 240
   Ile Tyr Thr Phe Asp Gly Ala Leu Asn Lys Ser Thr Val Pro Thr Asp
   65 70 75 80
   ttt agt agt gca aaa att gaa gtc tca caa tta cta aaa gga gat gcc 288
   Phe Ser Ser Ala Lys Ile Glu Val Ser Gln Leu Leu Lys Gly Asp Ala
   85 90 95
   tct ttg aag atg gat aag agt gat gct gtc tca cac aca gga aac tac 336
   Ser Leu Lys Met Asp Lys Ser Asp Ala Val Ser His Thr Gly Asn Tyr
   100 105 110
   act tgt gaa gta aca gaa tta acc aga gaa ggt gaa acg atc atc gag 384
   Thr Cys Glu Val Thr Glu Leu Thr Arg Glu Gly Glu Thr Ile Ile Glu
   115 120 125
   cta aaa tat cgt gtt gtt tca tgg ttt tct cca aat gaa aat tct aga 432
   Leu Lys Tyr Arg Val Val Ser Trp Phe Ser Pro Asn Glu Asn Ser Arg
   130 135 140
   gag aaa act cac aca tgc cca ccg tgc cca gca cct gaa ctc ctg ggg 480
   Glu Lys Thr His Thr Cys Pro Pro Cys Pro Ala Pro Glu Leu Leu Gly
   145 150 155 160
   gga ccg tca gtc ttc ctc ttc ccc cca aaa ccc aag gac acc ctc atg 528
   Gly Pro Ser Val Phe Leu Phe Pro Pro Lys Pro Lys Asp Thr Leu Met
   165 170 175
   atc tcc cgg acc cct gag gtc aca tgc gtg gtg gtg gac gtg agc cac 576
   Ile Ser Arg Thr Pro Glu Val Thr Cys Val Val Val Asp Val Ser His
   180 185 190
   gaa gac cct gag gtc aag ttc aac tgg tac gtg gac ggc gtg gag gtg 624
   Glu Asp Pro Glu Val Lys Phe Asn Trp Tyr Val Asp Gly Val Glu Val
   195 200 205
   cat aat gcc aag aca aag ccg cgg gag gag cag tac aac agc acg tac 672
   His Asn Ala Lys Thr Lys Pro Arg Glu Glu Gln Tyr Asn Ser Thr Tyr
   210 215 220
   cgg gtg gtc agc gtc ctc acc gtc ctg cac cag gac tgg ctg aat ggc 720
   Arg Val Val Ser Val Leu Thr Val Leu His Gln Asp Trp Leu Asn Gly
   225 230 235 240
   aag gag tac aag tgc aag gtc tcc aac aaa gcc ctc cca gcc ccc atc 768
   Lys Glu Tyr Lys Cys Lys Val Ser Asn Lys Ala Leu Pro Ala Pro Ile
   245 250 255
   gag aaa acc atc tcc aaa gcc aaa ggg cag ccc cga gaa cca cag gtg 816
   Glu Lys Thr Ile Ser Lys Ala Lys Gly Gln Pro Arg Glu Pro Gln Val
   260 265 270
   tac acc ctg ccc cca tcc cgg gat gag ctg acc aag aac cag gtc agc 864
   Tyr Thr Leu Pro Pro Ser Arg Asp Glu Leu Thr Lys Asn Gln Val Ser
   275 280 285
   ctg acc tgc ctg gtc aaa ggc ttc tat ccc agc gac atc gcc gtg gag 912
   Leu Thr Cys Leu Val Lys Gly Phe Tyr Pro Ser Asp Ile Ala Val Glu
   290 295 300
   tgg gag agc aat ggg cag ccg gag aac aac tac aag acc acg cct ccc 960
   Trp Glu Ser Asn Gly Gln Pro Glu Asn Asn Tyr Lys Thr Thr Pro Pro
   305 310 315 320
   gtg ctg gac tcc gac ggc tcc ttc ttc ctc tac agc aag ctc acc gtg 1008
   Val Leu Asp Ser Asp Gly Ser Phe Phe Leu Tyr Ser Lys Leu Thr Val
   325 330 335
   gac aag agc agg tgg cag cag ggg aac gtc ttc tca tgc tcc gtg atg 1056
   Asp Lys Ser Arg Trp Gln Gln Gly Asn Val Phe Ser Cys Ser Val Met
   340 345 350
   cat gag gct ctg cac aac cac tac acg cag aag agc ctc tcc ctg tct 1104
   His Glu Ala Leu His Asn His Tyr Thr Gln Lys Ser Leu Ser Leu Ser
   355 360 365
   ccg ggt aaa 1113
   Pro Gly Lys
   370
<210> 4
   <211> 371
   <212> PRT
   <213> Artificial
<220>
   <223> Fusion of CD47 part(1-142 amino acid) and Ig-Fc
<400> 4
   Met Trp Pro Leu Val Ala Ala Leu Leu Leu Gly Ser Ala Cys Cys Gly
   1 5 10 15
   Ser Ala Gln Leu Leu Phe Asn Lys Thr Lys Ser Val Glu Phe Thr Phe
   20 25 30
   Cys Asn Asp Thr Val Val Ile Pro Cys Phe Val Thr Asn Met Glu Ala
   35 40 45
   Gln Asn Thr Thr Glu Val Tyr Val Lys Trp Lys Phe Lys Gly Arg Asp
   50 55 60
   Ile Tyr Thr Phe Asp Gly Ala Leu Asn Lys Ser Thr Val Pro Thr Asp
   65 70 75 80
   Phe Ser Ser Ala Lys Ile Glu Val Ser Gln Leu Leu Lys Gly Asp Ala
   85 90 95
   Ser Leu Lys Met Asp Lys Ser Asp Ala Val Ser His Thr Gly Asn Tyr
   100 105 110
   Thr Cys Glu Val Thr Glu Leu Thr Arg Glu Gly Glu Thr Ile Ile Glu
   115 120 125
   Leu Lys Tyr Arg Val Val Ser Trp Phe Ser Pro Asn Glu Asn Ser Arg
   130 135 140
   Glu Lys Thr His Thr Cys Pro Pro Cys Pro Ala Pro Glu Leu Leu Gly
   145 150 155 160
   Gly Pro Ser Val Phe Leu Phe Pro Pro Lys Pro Lys Asp Thr Leu Met
   165 170 175
   Ile Ser Arg Thr Pro Glu Val Thr Cys Val Val Val Asp Val Ser His
   180 185 190
   Glu Asp Pro Glu Val Lys Phe Asn Trp Tyr Val Asp Gly Val Glu Val
   195 200 205
   His Asn Ala Lys Thr Lys Pro Arg Glu Glu Gln Tyr Asn Ser Thr Tyr
   210 215 220
   Arg Val Val Ser Val Leu Thr Val Leu His Gln Asp Trp Leu Asn Gly
   225 230 235 240
   Lys Glu Tyr Lys Cys Lys Val Ser Asn Lys Ala Leu Pro Ala Pro Ile
   245 250 255
   Glu Lys Thr Ile Ser Lys Ala Lys Gly Gln Pro Arg Glu Pro Gln Val
   260 265 270
   Tyr Thr Leu Pro Pro Ser Arg Asp Glu Leu Thr Lys Asn Gln Val Ser
   275 280 285
   Leu Thr Cys Leu Val Lys Gly Phe Tyr Pro Ser Asp Ile Ala Val Glu
   290 295 300
   Trp Glu Ser Asn Gly Gln Pro Glu Asn Asn Tyr Lys Thr Thr Pro Pro
   305 310 315 320
   Val Leu Asp Ser Asp Gly Ser Phe Phe Leu Tyr Ser Lys Leu Thr Val
   325 330 335
   Asp Lys Ser Arg Trp Gln Gln Gly Asn Val Phe Ser Cys Ser Val Met
   340 345 350
   His Glu Ala Leu His Asn His Tyr Thr Gln Lys Ser Leu Ser Leu Ser
   355 360 365
   Pro Gly Lys
   370

## Description

### Technical Field

The invention of this application relates to uses of a fusion protein of a CD47 partial peptide and an immunoglobin Fc fragment, and optionally an anti-SHPS-1 monoclonal antibody. More specifically, the invention of this application relates to uses of a fusion protein of a CD47 partial peptide and an immunoglobin Fc fragment, and optionally an anti-SHPS-1 monoclonal antibody capable of acting on a function of cell response mediated by SHPS-1.

### Background Art

It has been so far clarified that on a control mechanism of growth and adhesion of cells, minute crosstalk acts between both of them. For example, when the skin is damaged by injury or the like, cells of the skin begin to grow for covering a wound, and when cells are contacted with each other, the growth of cells stops (contact inhibition). In many cancer cells, this mechanism of contact inhibition is lost, which is considered to cause acquirement of indefinite growth activity or metastatic activity of cancer cells. For this reason, clarification of a mechanism of controlling growth and adhesion of cells is important in understanding life phenomenon such as organogenesis, and also important in understanding disorders such as metastasis of cancer cells.

In our country, the development of a new method for preventing or treating arteriosclerosis which often causes serious diseases has been an important issue in health medical care for persons of middle or advanced age. Recently, in a lesion of arteriosclerosis, a relation between immunocompetent cells, mainly macrophages and chronic inflammation has attracted much interest. Accordingly, it is quite important to clarify a minute control mechanism of macrophage functions on a molecular level for understanding the disease of arteriosclerosis in more detail and studying and developing more effective therapeutic strategies.

The present inventors have clarified that tyrosine phosphatase having an SH2 domain plays an important role in activating a Ras low-molecular G protein by stimulation of a growth factor (Non-patent Document 1). Further, they have found a receptor-type protein SHPS-1 (SH2-containing Protein Tyrosine Phosphatase Substrate-1) belonging to a immunoglobulin super family (Non-patent Document 2) or a docking protein Gab-1 as a dephosphorylation substrate protein of SHP-2. It has also been clarified that an SHPS-1/SHP-2 system or a Gab-1/SHP-2 system interacts with an integrin system which is a receptor molecule on a cell surface functioning in adhering cells to an extracellular matrix or a cadherin system being a molecular group necessary for adhering cells to each other, so that cell movement is also controlled through activation of an Rho low-molecular G protein.

The present inventors have lately obtained the results of study suggesting the possibility that SHPS-1 interacts with a receptor-type molecule CD47 as a physiological ligand of its extracellular region to form an intercellular signal transduction system (CD47-SHPS-1 system) (Non-patent Document 3). Further, CD47 is also expressed in vascular endothelial cells. Consequently, it is considered that CD47 of vascular endothelial cells interacts with SHPS-1 on macrophages to negatively control a macrophage function. It is considered that minute clarification of this CD47-SHPS-1 system can greatly contribute to elucidation of the foregoing "control mechanism of growth and adhesion" or "control mechanism of macrophage functions". As a result, it can be expected to contribute to, for example, inhibition of metastasis of cancer cells expressing SHPS-1 and prevention or treatment of arteriosclerosis.
Non-patent Document 1: Noguchi, T., Matozaki, T., et al.: Mol. Cell. Biol., 14: 6674-6682, 1994
Non-patent Document 2: Fujioka, Y., Matozaki, T., et al.: Mol. Cell. Biol., 16: 6887-6899, 1996
Non-patent Document 3: Yamao, T., Noguchi, T., et al.: J. Biol. Chem., 277: 39833-39839, 2002

Seiffert et al. (Blood, Vol 94, No 11, December 1 1999, pages 3633-3643) discloses that human signal-regulatory protein is expressed on normal, but not on subsets of leukemic myeloid cells and mediates cellular adhesion involving its counter receptor CD47.

WO 02/092784 A2 concerns polypeptides that can modulate SIRPα-CD47 functions and methods of use of the polypeptides are presented.

Although a possibility that the CD47-SHPS-1 system is involved in "control mechanism of growth and adhesion" and "control mechanism of macrophage functions" is suggested and these are considered to be applied to prevention or treatment of various diseases, CD47 is a membrane penetration-type protein and it is difficult to allow it to act as a ligand in view of time and quantitative determination. Thus, there was no effective method for controlling functions of SHPS-1.

In production of an anti-SHPS-1 antibody, a base sequence encoding SHPS-1 as an immunogen at this time has been identified for the first time by the present inventors (GenBank Accession No.: JC5287 (protein), GenBank Accession No.: E15703A (nucleic acid) Yamamoto, T., et al., Biochem. Biophys. Res. Commun., 231: 61-67, 1997). However, they have not come to produce the antibody.

Under the foregoing circumstances, the invention of this application has been made, and it aims to provide, upon solving the existing problems, the use of a fusion protein of a CD47 partial peptide and an immunoglobin Fc fragment and optionally an anti-SHPS-1 monoclonal antibody capable of acting on a function of cell response mediated by SHPS-1.

### Disclosure of the Invention

The invention of this application provides, as a means for solving the foregoing problems, an *in vitro or ex vivo* method to inhibit movement of cells by controlling a function of cell response mediated by SHPS-1, which comprises contacting SHPS-1 on the cell with a fusion protein of a CD47 partial peptide and an immunoglobulin Fc fragment, which fusion protein has an amino acid sequence of SEQ ID No. 4;

The invention further provides a fusion protein of a CD47 partial peptide and an immunoglobulin Fc fragment, which has an amino acid sequence of SEQ ID No. 4, for use in a method for the treatment of cancers expressing SHPS-1, wherein the treatment is achieved by inhibiting metastasis of cancer cells.

Said CD47 partial peptide has an amino acid sequence constituting an extracellular region having an immunoglobulin-like structure of a CD47 protein and which can specifically bind to an N-terminal immunoglobulin-like structure of a dephosphorylation substrate protein SHPS-1 of an SH2 domain-containing protein.

Said CD47 partial peptide has the amino acid sequence of SEQ ID No. 2.

The CD47 partial peptide used in accordance with the invention has an immunoglobulin Fc fragment bound and has an amino acid sequence of SEQ ID No. 4, which is optionally an expression product of a polynucleotide having a base sequence of SEQ ID No. 3.

Also disclosed herein is a polynucleotide having a base sequence of SEQ ID No. 3, and
an expression vector carrying said polynucleotide.

Also disclosed herein is a transformant cell obtained with said expression vector.

The method and use of the present invention may further comprise addition of an anti-SHPS-1 monoclonal antibody which specifically recognizes and is capable of binding to a dephosphorylation substrate protein SHPS-1 of an SH2 domain-containing protein.

Also disclosed herein is a hybridoma cell strain producing said antibody.

Also disclosed herein is a composition characterized by containing, as an active ingredient, at least one of the CD47 partial peptide as described herein and the anti-SHPS-1 monoclonal antibody along with a pharmacological component.

### Brief Description of the Drawings

Fig. 1 is a schematic view showing a state of adding a CD47 protein and/or an anti-SHPS-1 monoclonal antibody to SHPS-1 expressed in melanoma cells.
Fig. 2 is a schematic view showing CD47-Fc produced by fusing an extracellular region of 5-time transmembrane-type CD47 present on a cell membrane with IG-Fc.
Fig. 3 is a schematic view showing a mechanism of inhibiting cell movement in a CD47-SHPS-1 system by addition of CD47-Fc.
Fig. 4 is graphs showing confirmation of expression of SHPS-1 and CD47 in melanoma cells (WM239a). (A) shows results of confirming SHPS-1 expression, and (B) shows results of confirming CD47 expression.
Fig. 5 is a graph showing an effect of CD47-Fc and an anti-SHPS-1 monoclonal antibody on inhibiting cell movement.
Fig. 6 is a graph showing an effect on inhibiting cell movement in case of further adding a secondary antibody to the anti-SHPS-1 monoclonal antibody.
Fig. 7 is a graph showing an effect of the anti-SHPS-1 monoclonal antibody on inhibiting cell movement in CHO-Ras cells expressing SHPS-1.
Fig. 8 is a graph showing an inhibitory effect of the anti-SHPS-1 monoclonal antibody on CHO-IR cells expressing SHPS-1 and variant CHO-Ras cells not expressing SHPS-1.
Fig. 9 is graphs showing a platelet life in a wild-type mouse (WT) and an SHPS-1-deficient-type mouse (KO). (A) shows the number of days after administration of an anticancer agent, and (B) its half-life by biotin labeling.
Fig. 10 is a view showing the increase in phagocytic activity of macrophages. (A) shows wild-type macrophages (WT), (B) SHPS-1-deficient-type macrophages (KO), and (C) a graph that compares a wild type (WT) with a SHPS-1-deficient type (KO).

### Best Mode for Carrying Out the Invention

The invention of this application has the foregoing characteristics, and embodiments thereof are described in detail below.

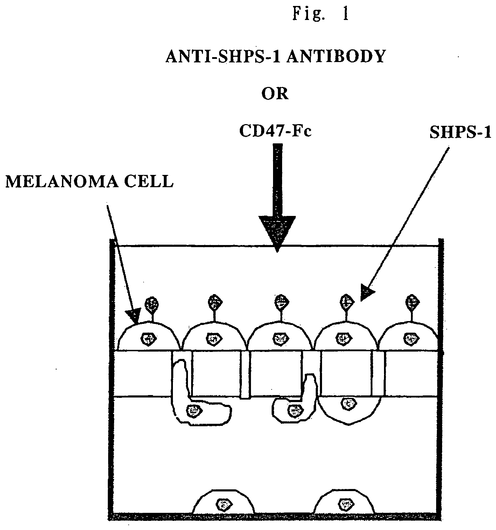

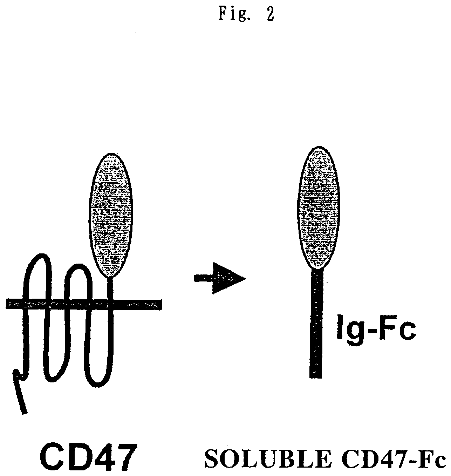

Since the CD47 protein disclosed herein (for example, GenBank Accession No.: BT00697) was identified as a molecule associated with an integrin being a cell adhesion molecule, it is also called IAP (Integrin-Associated Protein), and is a five-time transmembrane-type protein universally expressed in tissues. As shown in Figs. 1 and 2, its extracellular region has one immunoglobulin-like structure which can bind to an immunoglobulin-like structure present on an N-terminal side of SHPS-1.

The CD47 partial peptide of the fusion protein used in accordance with the method and use of the invention is a polypeptide which has an amino acid sequence constituting an extracellular region having an immunoglobulin-like structure of a CD47 protein and which is specifically bound to an N-terminal immunoglobulin-like structure of a dephosphorylation substrate protein SHPS-1 of an SH2 domain-containing protein. The amino acid sequence of SEQ ID No. 2 disclosed herein, is an amino acid sequence constituting the extracellular region having the immunoglobulin-like structure of the CD47 protein. This CD47 partial peptide is a partial sequence (1st to 142nd amino acids) of a known CD47 protein (for example, GenBank Accession No.: BT00697), and it can be cloned from a human cell-derived cDNA library. The cDNA library can be synthesized by a known method (for example, Okayama, H., et al., Mol. Cell. Biol. 2; 161-170, 1982) using poly(A)+RNA extracted from human cells or the like as a template, and a commercially available cDNA library can also be used.

The CD47 partial peptide can be cloned from the cDNA library by synthesizing an oligonucleotide on the basis of a base sequence of any portion in the CD47 protein, as described above, and conducting screening through colony hybridization or plaque hybridization by a known method using this oligonucleotide as a probe. Further, a CD47 partial peptide cDNA can be obtained by synthesizing an oligonucleotide to be hybridized on both terminals of a desired polynucleotide and conducting RT-PCR from mRNA isolated from cells using this as a primer. A polynucleotide encoding an amino acid sequence of SEQ ID No. 2 (namely, a polynucleotide having a base sequence of SEQ ID No. 1) can be prepared by digesting this cDNA with an appropriate restriction enzyme.

The method and use of the invention of this application use a fusion protein (hereinafter sometimes referred to as CD47-Fc) which may be obtained by binding a CD47 partial peptide to an immunoglobulin Fc fragment (hereinafter sometimes referred to as Ig-Fc), which may result from digestion of an antibody with a protease such as papain. Said fusion protein has an amino acid sequence of SEQ ID No. 4 which is an amino acid sequence encoding CD47-Fc, a fusion protein of the CD47 partial peptide and Ig-Fc. Said fusion protein is optionally an expression product of a polynucleotide having a base sequence of SEQ ID No. 3. Also disclosed herein is the polynucleotide having the base sequence of SEQ ID No. 3.

The CD47 partial peptide constituting CD47-Fc having the amino acid of SEQ ID No. 4 encodes 1st to 142nd amino acids, and Ig-Fc encodes 143rd to 371st amino acids.

Since the above-obtained CD47-Fc is soluble, antibody-like and divalent, an SHPS-1 crosslinking function is obtained, which is more preferable for realizing an effect of inhibiting cell movement or the like.

Further, an effect of CD47-Fc or an anti-SHPS-1 monoclonal antibody on accumulation and crosslinking of SHPS-1 is enhanced by addition of a secondary antibody to increase an inhibitory effect mediated by a function of SHPS-1. This secondary antibody is an IgG antibody to antibody series of a primary antibody, and it is not particularly limited. For example, when it is an antibody obtained by immunizing a mouse, an antibody is a mouse IgG, and an anti-mouse IgG antibody is therefore used as a secondary antibody. Further, in case of a human Fc fragment protein, an anti-human IgGFc antibody is used.

The expression vector for the fusion protein used in accordance with the method and use of the invention may be obtained by treating a polynucleotide encoding the CD47 partial peptide and a polynucleotide encoding Ig-Fc respectively with appropriate restriction enzymes and binding them through ligation and introducing the resulting fragment (namely, a polynucleotide comprising a base sequence of SEQ ID No. 3). CD47-Fc may be expressed using this expression vector. Examples of the available expression vector can include pTracerCMV, pKA1, pCDM8, pSVK3, pMSG, pSVL, pBK-CMV, pBK-RSV, EBV vector, pRS, pcDNA3, pMSG, pYES2 and the like.

Also disclosed herein, the CD47 partial peptide can be utilized as a structure (monovalent) of the CD47 partial peptide only. Disclosed herein, it is possible that the Ig-Fc fragment is formed into a fusion protein by arbitrarily employing another peptide (for example, His-tag (histidine-tag) or GST (glutathione-S-transferase)).

The fusion protein used in accordance with the method and use of the present invention may be obtained by transforming the expression vector carrying the foregoing polynucleotide comprising the base sequence of SEQ ID NO. 3 in any culture of cells. The cultured cells are transformant cells that stably express CD47-Fc, and they have been established by selective culture in a cell culture medium containing various drugs (for example, G418) adapted to drug resistance genes that the expression vector carries. CD47-Fc can be obtained by recovering and purifying a cell supernatant of the transformant cell strain. The culture cells are not particularly limited, and various cells including Hela cells, COS7 cells and CHO cells can be used.

In a method for recovering cells, generally, cells are recovered from an incubator along with a medium, the medium containing the cells is treated by low-speed centrifugation, and the cells precipitated are suspended in an appropriate buffer solution. Examples of the buffer solution can include a Tris buffer solution, a HEPES buffer solution, a phosphate buffer solution and the like. The buffer solution can properly be selected according to the type of cells and the experimental purpose. The cell suspension can be mixed, as required, with a cell storage solution such as DMSO or a commercial storage solution kit (for example, Cellvation™; manufactured by Celox Laboratories) to store the mixture in liquid nitrogen or a freezer of not higher than -80° for a long period of time.

As a method for purifying an intended expressed protein such as CD47-Fc, the cell supernatant can be purified by employing, for example, affinity column or gel filtration. Especially in case of CD47-Fc fusion protein used in accordance with the method and use of the invention of this application, the fusion protein with the Fc fragment is formed. Accordingly, an affinity column using a Protein-A agarose having high to the Fc fragment is preferably utilized.

The origin of the CD47 partial peptide constituting CD47-Fc or Ig-Fc is preferably mammals. Examples thereof can include a sheep, a goat, a rat, a mouse, a rabbit, a horse, cattle, humans and the like. Humans are especially preferable for the purpose of inhibition of cancer cell metastasis of humans, prevention or treatment of arteriosclerosis of humans or the like.

CD47-Fc can be bound to SHPS-1 as a type of a ligand of SHPS-1, and a function of SHPS-1 can be controlled by adjusting the use amount, the use timing or the like of CD47-Fc at this time. This SHPS-1 is a receptor-type protein as a dephosphorylation substrate protein of a tyrosine phosphatase SHP-2 having an SH2 domain. It is a protein having three immunoglobulin-like structures in an extracellular region and carrying plural tyrosine residues that undergo phosphorylation in an intracellular region.

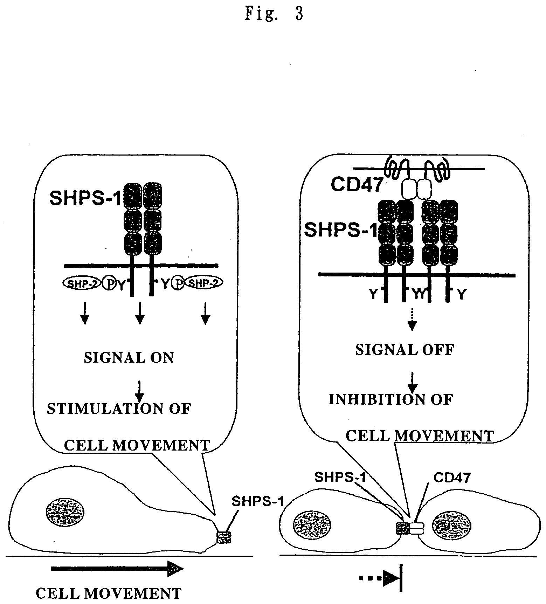

It is considered, as stated above, that in CD47-Fc, an immunoglobulin-like structure on an N-terminal side of SHPS-1 and one immunoglobulin-like structure in a CD47 extracellular region are bound to provide interaction with SHPS-1 (namely, a CD47-SHPS-1 system). This CD47-SHPS-1 system is involved in negative control of universal cell movement. As shown in Fig. 3, it is considered that the CD47-SHPS-1 system inhibits cell movement bidirectionally by contact of cells expressing a CD47 molecule and an SHPS-1 molecule respectively and that the CD47-SHPS-1 system is greatly involved in a molecular mechanism of contact inhibition.

The anti-SHPS-1 monoclonal antibody which is optionally additionally used in accordance with the method and use of the invention of this application is an anti-SHPS-1 monoclonal antibody which specifically recognizes and is capable of binding to a dephosphorylation substrate protein SHPS-1 of an SH2 domain-containing protein. Accordingly, the function of SHPS-1 can be controlled, similarly to CD47-Fc, by adjusting the use amount, the use timing or the like of the anti-SHPS-1 monoclonal antibody.

In the anti-SHPS-1 monoclonal antibody, SHPS-1 which is its target is preferably mammalian. Examples thereof include a sheep, a goat, a rat, a mouse, a rabbit, a horse, cattle, humans and the like. In view of the purpose of inhibition of cancer cell metastasis of humans, prevention or treatment of arteriosclerosis of humans or the like, humans are especially preferable similarly to the CD47 partial peptide.

The anti-SHPS-1 monoclonal antibody which may optionally additionally be used in accordance with the method and use of the invention can be produced from the hybridoma cell strain disclosed herein according to a prescribed method. First, a peptide SHPS-1 (GenBank Accession No.: JC5287 (protein), GenBank Accession No.: E15703A (nucleic acid), Yamamoto, T., et al., Biochem. Biophys. Res. Commun., 231: 61-67, 1997 and etc) can be obtained by purifying this SHPS-1 through immunoprecipitation using an Fc specific antibody. It may be produced in Escherichia coli, yeast or the like as a fusion protein with GST (glutathione-S-transferase) or as a fusion protein with immunoglobulin Fc. The resulting SHPS-1 is administered to an animal to be immunized (for example, a mouse, a rat or a rabbit) as an immunogen along with any carrier (for example, bovine serum albumin (FCS)) for immunization. As required, additional immunization is properly conducted after a prescribed period of time to fully sensitize this immunized animal. Subsequently, antibody-productive cells such as lymphocytes, prepared from the spleen or the lymph node of the immunized animal are fused with myeloma cells according to a known method (for example, a method for cell fusion in a solution of a polymer such as polyethylene glycol at a high concentration or a method for cell fusion by electric stimulation) to prepare a hybridoma cell strain that produces an anti-SHPS-1 monoclonal antibody, and its cell supernatant is purified. In this manner, the anti-SHPS-1 monoclonal antibody can be obtained. ("Tan Kuron Kotai", Iwasaki Tatsuo, Ando Tamie, et al., Kodansha, 1987; Seiffert, M., et al., Blood 94; 3633-3643, 1999, Seiffert, M., et al., Blood 97; 2741-2749, 2001, and the like). The myeloma cells are not particularly limited. In consideration of convenience in selecting a hybridoma from fusion cells, an HGPRT (Hypoxanthine-guanine Phosphoribosyl Transferase)-deficient strain in which the selection method has been established is preferably used. Examples thereof can include X63-AG8(X63), NS-1Ag4/1(NS-1), P3X63-AG8.UI (P3UI), X63-Ag8.653(X63.653), SP2/0-AG14(SP2/0), Fo and the like which are derived from mice.

Fusion cells and non-fusion cells can be selected by, for example, a known HAT (hypoxanthine·aminopterin·thymidine) selection method. This method is a method which is effective in case of producing fusion cells using myeloma cells of an HGPRT-deficient strain that cannot be alive in the presence of aminopterin. In this method, only fusion cells having resistance to aminopterin can selectively be cultured by culturing fusion cells and non-fusion cells in a HAT medium.

The screening of hybridoma cells producing the desired monoclonal antibody can be conducted by a known method such as EIA (enzyme immunoassay), RIA (radioimmunoassay) or a fluorescent antibody method.

The hybridoma cell strain obtained in the foregoing manner can be stored, similarly to the CD47-Fc production cell strain, in liquid nitrogen or a freezer of not higher than -80°C by being mixed with a cell storage solution such as DMSO or a storage liquid kit (for example, Cellvation™; manufactured by Celox Laboratories) as required.

The fusion protein used in accordance with the use of the present invention can be in a composition optionally containing, as an active ingredient, the anti-SHPS-1 monoclonal antibody, along with a pharmacological component. This composition can be applied to inhibition of cancer cell metastasis, prevention or treatment of arteriosclerosis and the like. Said composition may contain said fusion protein and the anti-SHPS-1 monoclonal antibody along with the pharmacological component.

The "pharmacological component" first means various carriers used in the ordinary drug production. The "carrier" can properly be selected from the wide range according to the type of target diseases or the dosage form of the drug. Said composition is preferably in a unit dosage form capable of oral administration or administration by injection. Especially, in case of administration by injection, local injection, intraperitoneal administration, selective intravenous injection, intravenous injection, subcutaneous injection, organ perfusate injection and the like can be employed.

Oral liquid preparations such as a suspension and a syrup can be produced using water, saccharides such as sucrose, sorbitol and fructose, glycols such as polyethylene glycol, oils such as sesame oil and soybean oil, preservatives such as alkyl p-hydroxybenzoate, flavors such as a strawberry flavor and a peppermint, and the like.

Powders, pills, capsules and tablets can be formed using excipients such as lactose, glucose, sucrose and mannitol, disintegrants such as starch and sodium alginate, lubricants such as magnesium stearate and talc, binders such as polyvinyl alcohol, hydroxypropyl cellulose and gelatin, surfactants such as a fatty acid ester, plasticizers such as glycerin, and the like. Tablets and capsules are preferably in a unit dosage form in the composition used in this invention because of easy administration. In producing tablets or capsules, solid production carriers are used.

An injection solution can be formed using a carrier comprising a salt solution, a glucose solution or a mixture of salt water and a glucose solution, various buffer solutions and the like. It is also possible that formulation is conducted in a powdery state, and the powder is mixed with the liquid carrier in use to prepare an injection solution.

The pharmacological component is secondly a component for rendering the CD47-Fc fusion protein and/or the anti-SHPS-1 monoclonal antibody in a dosage form introducible into cells. For example, a composition can be formed by mixing the polypeptide with a pharmacologically acceptable solution without changing the structure or the function of the polypeptide. Such a composition can be introduced into target cells by a method for introduction into cells via microinjection, a method for introduction into cells using lipids or the like.

The invention of this application is specifically described in more detail below by referring to Examples. Of course, the invention of this application is not limited by the following Examples.

### Examples

### Example 1: Verification on an effect of inhibiting cell movement

### 1. Production of antibodies

### (i) Anti-CD47 monoclonal antibody

The anti-CD47 monoclonal antibody used in this Example was produced as an anti-human CD47 monoclonal antibody (CC2C6), and this was utilized.

With respect to a production process, production and purification were conducted by a known method ("Tan Kuron Kotai", Iwasaki Tatsuo, Ando Tamie, et al., Kodansha, 1987; Seiffert, M., et al. Blood 94; 3633-3643, 1999, Seiffert, M., et al. Blood 97; 2741-2749, 2001, and the like).

Specifically, a CD47 protein was administered to a mouse as an immunogen along with bovine serum albumin (FCS)) as a carrier for immunization. As required, additional immunization was properly conducted after a prescribed period of time to fully sensitize the mouse. Antibody-productive cells such as lymphocytes, produced from the spleen or the lymph node of this mouse were fused with mouse myeloma cells P3U according to a known method to produce a CD47 antibody-productive hybridoma cell strain, and the cell supernatant was purified. In this manner, the anti-CD47 monoclonal antibody was obtained.

### (ii) Anti-SHPS-1 monoclonal antibody

An anti-SHPS-1 monoclonal antibody (SE12C3) was produced and purified by the same process as in (i) except that a peptide SHPS-1 was used as an immunogen.

This SHPS-1 was obtained by conducting purification through immunoprecipitation using an Fc specific antibody.

### 2. Confirmation of expression of SHPS-1 and CD47 in melanoma cells

Expression of CD47 and SHPS-1 was confirmed by an immunofluorescence labeling analysis method using human malignant melanoma cells WM239a (M. Herlyn (provided by Wistar Institute, Philadelphia, PA)) as melanoma cells.

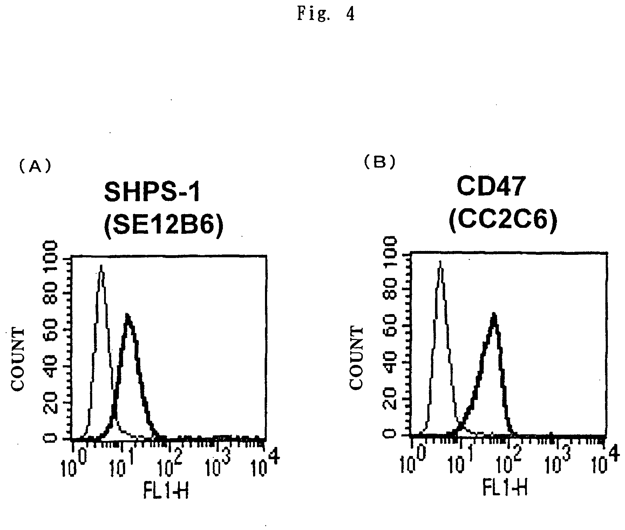

Consequently, as shown in Fig. 4, it was confirmed that expression was observed in both of SHPS-1 and CD47. In (A), an anti-SHPS-1 monoclonal antibody (SE12C3) was incubated with WM239a, and in (B), an anti-CD47 monoclonal antibody (CC2C6) was incubated with WM239a. After the incubation, detection was conducted by using an FITC-labeled anti-mouse IgG polyclonal antibody and by flow cytometry (thick line). A negative control was provided by replacing the anti-SHPS-1 monoclonal antibody or the anti-CD47 monoclonal antibody with a normal mouse IgG (thin line).

### 3. Production of CD47-Fc

A cDNA of human CD47 was obtained by being provided from Mr. F. P. Lindberg (Washington University, St Louis, MO).

First, a cDNA encoding an Fc protein of human IgG1 was obtained by a known method (Liu, Y. C., et al., Proc. Natl. Acad. Sci. 90; 8957-8961, 1993) using PCR. This PCR product was digested with SpeI and NotI. pEFneo vector (manufactured by Invitrogen) was ligated in sites homologous to SpeI and NotI to produce pEFneoFc76.

Subsequently, a cDNA encoding a human CD47 partial peptide (1 to 142 amino acids) was obtained by PCR using a cDNA of a full-length human CD47 protein as a template. At this time, 5'-TAATACTAGTCTGCTGCTCCAGAC ACCTGCG-3' was used as a sense primer, and 5'-TAGCTCTAGAATTTTCA TTTGGAGAAAACCATGAAAC-3' as an antisense primer. The resulting PCR product was digested with XbaI and SpeI, and introduced into a SpeI site of pEFneoFc76. The resulting vector was digested with PstI and NotI, and the thus-obtained fragment was introduced into positions homologous to PstI and NotI sites of pTracerCMV to form plasmid pTracerCMV-hCD47-Fc. pTracerCMV-hCD47-Fc was transfected with CHO-Ras cells, and selective culture was conducted according to a prescribed method using Zeocin. Judgement of a cell strain that expresses and secretes the CD47-Fc fusion protein was performed by an immunoblotting method using an anti-human IgG polyclonal antibody in a cell supernatant. In the purification of CD47-Fc, the supernatant was purified by column chromatography with rProtein A-Sepharose 4FF (manufactured by Amersham Pharmacia Biotech).

### 4. Action of anti-SHPS-1 monoclonal antibody and CD47-Fc on WM239a

The action on malignant melanoma cells WM239a was examined by measurement of cell movement. The cell movement was measured by a known method using a Transwell apparatus (manufactured by Corning) (Longo, N., et al., Blood 98; 3717-3726, 2001).

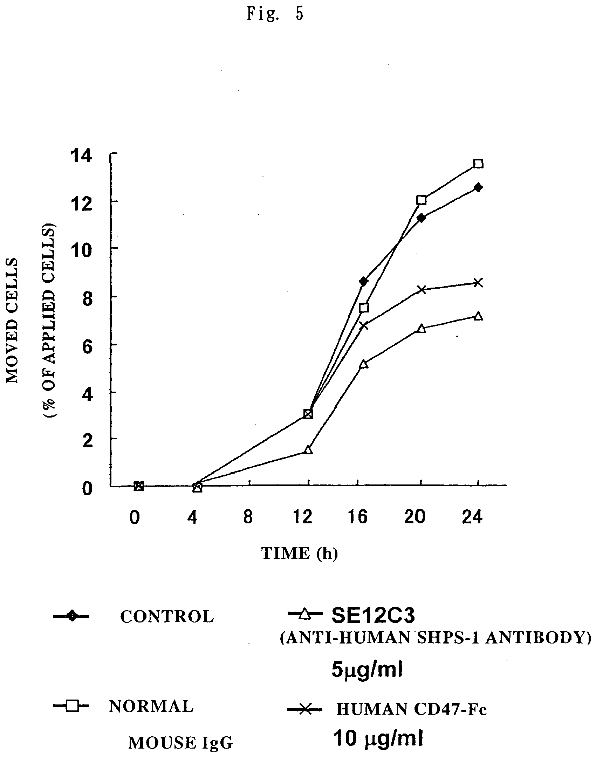

As shown in Fig. 5, an effect of inhibiting cell movement of malignant melanoma cells WM239a was confirmed in both of the anti-SHPS-1 monoclonal antibody and CD47-Fc. That is, the SHPS-1 ligand bound to CD47 (namely, anti-SHPS-1 monoclonal antibody and CD47-Fc) can inhibit cell movement, and inhibition of metastasis of cancer cells can be expected.

### 5. Effect provided by addition of a secondary antibody

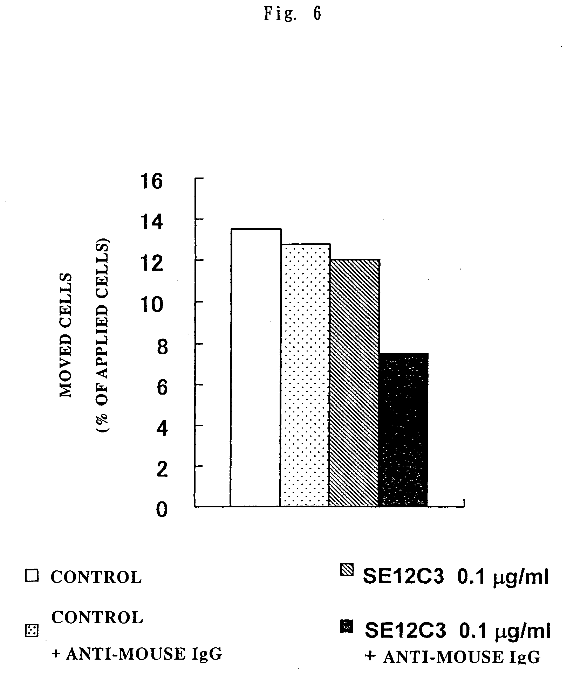

With respect to the effect of inhibiting cell movement in 4, it was confirmed, as shown in Fig. 6, that inhibition of cell movement was enhanced by addition of a secondary antibody.

It is considered that an effect of the fused protein CD47-Fc or the anti-SHPS-1 monoclonal antibody on SHPS-1 accumulation and crosslinking is enhanced to thereby increase an inhibitory effect mediated by the activity of SHPS-1.

An anti-mouse IgG was used as a secondary antibody in this Example.

### 6. Action of an anti-SHPS-1 monoclonal antibody on CHO-Ras cells

An action on CHO-Ras cells (provided by S. Shirahata (Kyushu University, Fukuoka, Japan)) stably expressing H-Ras was examined by measurement of cell movement. The measurement of cell movement was conducted, as in 4, by a known method using a Transwell apparatus (manufactured by Corning) (Longo, N., et al., Blood 98; 3717-3726, 2001).

In this Example, examination was conducted using two types of the anti-SHPS-1 monoclonal antibody, an anti-human SHPS-1 monoclonal antibody (SE12C3) and an anti-rat SHPS-monoclonal antibody (2F34).

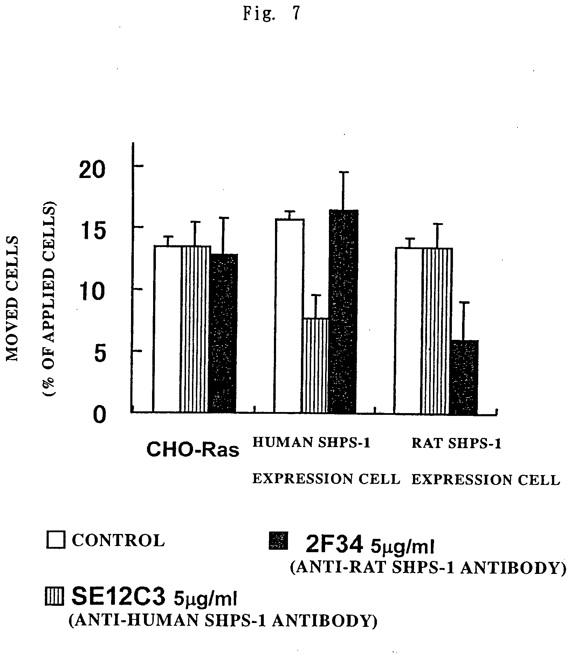

As shown in Fig. 7, an effect of inhibiting cell movement of CHO-Ras cells was confirmed in both of the anti-human SHPS-1 monoclonal antibody (SE12C3) and the anti-rat SHPS-1 monoclonal antibody (2F34). Especially, it was confirmed that the anti-human SHPS-1 monoclonal antibody acted more effectively on human SHPS-1 expression cells, whereas the anti-rat SHPS-1 monoclonal antibody acted more effectively on rat SHPS-1 expression cells. Incidentally, normal mouse IgG was used as a control.

### 7. Examination of necessity for an SHP-2 binding motif using CHO-IR cells

With respect to CHO-IR cells stably expressing a human insulin receptor, CHO-IR-SHPS-1-WT cells carrying a rat SHPS-1 normal type and CHO-IR-rSHPS-1-4F cells carrying a rat SHPS-1 variant type with all four tyrosine residues substituted by phenylalanine residues were used. Further, as an antibody, two types, an anti-rat SHPS-1 monoclonal antibody (2F34) and an anti-rat SHPS-1 monoclonal antibody (9F10) were used.

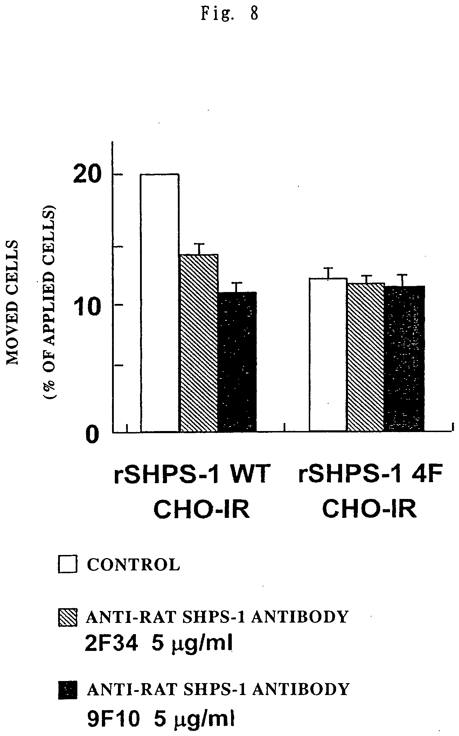

Consequently, as shown in Fig. 8, in CHO-IR-rSHPS-1-4F cells, no difference was found among the control, the anti-rat SHPS-1 monoclonal antibody (2F34) and the anti-rat SHPS-1 monoclonal antibody (9F10). Meanwhile, in CHO-IR-SHPS-1-WT cells, no effect was found in the control. However, the inhibitory effect was confirmed in the anti-rat SHPS-1 monoclonal antibody (2F34) and the anti-rat SHPS-1 monoclonal antibody (9F10). That is, it can be understood that the SHP-2 binding motif of SHPS-1 is necessary for inhibition of cell movement with the anti-SHPS-1 monoclonal antibody.

### Example 2: Verification in a macrophage function

### 1. Change in number of peripheral blood platelets in an SHPS-1 gene-deficient mouse

A gene-deficient mouse which is deficient in an intracellular region of SHPS-1 (hereinafter sometimes referred to as SHPS-1 KO mouse) was produced according to a known method (for example, supervised by Nomura Tatsuji, compiled by Kachiki Gen, et al., Hassei Kogaku Jikken Manuaru, Kodansha Scientific, 1987, Capecchi, MR., Science 244: 1288-1292, 1989, and the like).

The peripheral blood of this SHPS-1 KO mouse was analyzed by flow cytometry using each blood cell ingredient specific antibody. Then, the number of platelets was reduced by up to about 70% in comparison with a wild-type mouse (Table 1).

**Table 1**

| | | +/- male | -/- male | +/- female | -/- female |
|---|---|---|---|---|---|
| RBC | (x10⁴/ml) | 925+/-25 | 924+/-20 | 973+/-27 | 932+/-8 |
| WBC | (x10²/ml) | 14.6+/-1.4 | 25.0+/-1.6 | 23.2+/-1.8 | 17.2+/-0.7 |
| Hb | (g/dL) | 15.7+/-0.33 | 14.8+/-0.44 | 16.6+/-0.54 | 15.7+/-0.26 |
| MCV | (fL) | 57.6+/-0.67 | 54.6+/-0.68 | 57.2+/-0.53 | 55.6+/-0.57 |
| PLT | (x10⁴/ml) | 98.2+/-4.1* | 75.6+/-3.5* | 64.3+/-4.3* | 64.9+/-2.3* |
| MPV | (fL) | 5.56+/-0.07 | 5.76+/-0.05 | 5.70+/-0.12 | 5.52+/-0.13 |

| | | | | | |
|---|---|---|---|---|---|
| *Student's test, P< .005 | | | | | |

It was confirmed that the change in number of platelets was observed in 4 weeks from birth and continued for at least 13 weeks.

### 2. Verification of a cause of decrease in number of platelets

With respect to verification of a cause of decrease in number of platelets, growth activity and differentiation activity of megakaryocytes obtained from an SHPS-1 KO mouse were examined by in-vitro culture, and recovery of number of platelets after administration of an anticancer agent 5FU was conducted with a flow cytometer.

Specifically, regarding megakaryocyte colony formability in the femur, MGDF as its growth stimulation factor was administered, and the reaction was examined. However, no difference was found between a wild-type mouse and an SHPS-1 KO mouse.

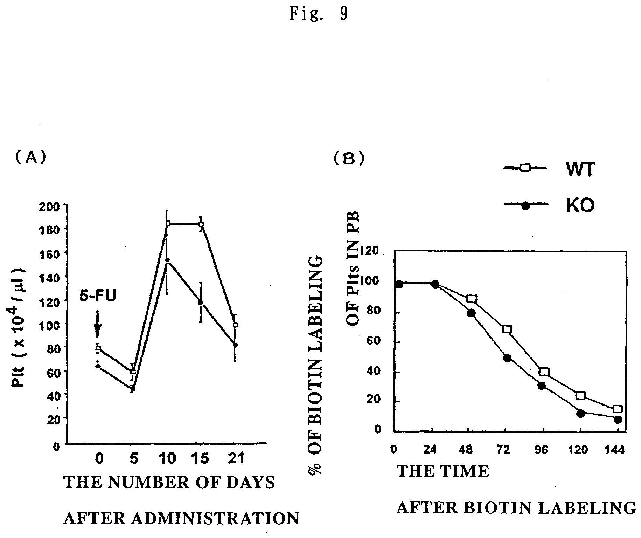

Meanwhile, in the platelet life, a recovery process of the number of platelets after inhibition of myeloma by administration of 5-FU as an anticancer agent was analyzed. Consequently, as shown in Fig. 9 (A), the number of platelets after 15 days from the administration was 182 ± 13.7 x 10⁴/µl in the wild-type mouse (WT), whereas it was 117 ± 41.2 x 10⁴/µl in the SHPS-1 KO mouse (KO) to find a difference. Further, in Fig. 9(B), an in-vivo half-life of biotin-labeled platelets was measured. Consequently, the half-life of the SHPS-1 KO mouse (KO) was confirmed to be significantly shortened in comparison with that of the wild-type mouse (WT). Thus, although hindrance of recovery with 5FU was observed, the result of the increase in disappearance speed of biotinized platelets was confirmed. Thus, it was identified that it does not mean hindrance in production process but increase in consumption process.

### 3. Verification of a mechanism of participation in decrease in number of platelets in SHPS-1 KO mouse

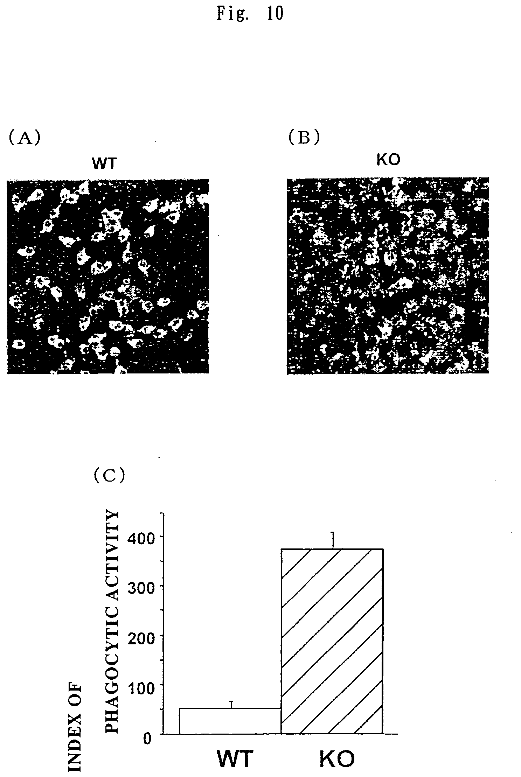

Intraperitoneal macrophages were isolated from an SHPS-1 KO mouse by thioglycote intraperitoneal injection, and in-vitro phagocytic activity was analyzed. Phagocytic activity of mouse red cells was examined using the macrophages. Then, as shown in Figs. 10 (A), (B) and (C) respectively, significant enhancement of phagocytic activity to platelets was observed in macrophages derived from the SHPS-1 KO mouse (KO) in comparison with macrophages derived from the wild-type mouse (WT). From this fact, it can be understood that the decrease in number of platelets and shortening of life of platelets are attributable to the phagocytic activity of macrophages. That is, CD47 on platelets is bound to SHPS-1 of macrophages to negatively control the phagocytosis process of platelets by macrophages. That is, the anti-SHPS-1 monoclonal antibody as a ligand of SHPS-1 or CD47-Fc acts to improve the phagocytic activity of macrophages to platelets involved in blood coagulation or the like, which can be applied to treatment of arteriosclerosis or the like.

### Industrial Applicability

As has been thus far described in detail, the CD47 partial peptide and the anti-SHPS-1 monoclonal antibody which can act on the function of cell response mediated by SHPS-1 to contribute to inhibition of cancer cell metastasis, improvement of arteriosclerosis or the like are provided.

## Claims

1. An *in vitro* or *ex vivo* method to inhibit movement of cells by controlling a function of cell response mediated by SHPS-1, which comprises contacting SHPS-1 on the cell with a fusion protein of a CD47 partial peptide and an immunoglobulin Fc fragment, which fusion protein has an amino acid sequence of SEQ ID No. 4.

2. The method of claim 1, which further comprises the addition of an anti-SHPS-1 monoclonal antibody which specifically recognizes and is capable of binding to SHPS-1.

3. The method of claim 1 or 2, wherein the method is used to inhibit movement of cancer cells.

4. A fusion protein of a CD47 partial peptide and an immunoglobulin Fc fragment, which has an amino acid sequence of SEQ ID No. 4 for use in a method for the treatment of cancers expressing SHPS-1, wherein the treatment is achieved by inhibiting metastasis of cancer cells.

5. The fusion protein for use according to claim 4, which is in a composition which further contains, as an active ingredient, an anti-SHPS-1 monoclonal antibody which specifically recognizes and is capable of binding to SHPS-1.

## Patentansprüche

1. In-vitro- oder Ex-vivo-Verfahren zum Hemmen der Bewegung von Zellen durch das Kontrollieren einer Funktion der SHPS-1-vermittelten Zellantwort, das das Inkontaktbringen von SHPS-1 auf der Zelle mit einem Fusionsprotein eines CD47-Teilpeptids und einem Immunoglobulin-Fc-Fragment umfasst, wobei das Fusionsprotein eine Aminosäuresequenz der SEQ ID Nr. 4 aufweist.

2. Verfahren nach Anspruch 1, das ferner die Zugabe eines monoklonalen Anti-SHPS-1-Antikörpers umfasst, der SHPS-1 spezifisch erkennt und fähig ist, daran zu binden.

3. Verfahren nach Anspruch 1 oder 2, wobei das Verfahren verwendet wird, um die Bewegung von Krebszellen zu hemmen.

4. Fusionsprotein eines CD47-Teilpeptids und Immunoglobulin-Fc-Fragment, das eine Aminosäuresequenz der SEQ ID Nr. 4 aufweist, zur Verwendung in einem Verfahren zur Behandlung von Krebsformen, die SHPS-1 exprimieren, wobei die Behandlung erreicht wird, indem die Metastasenbildung von Krebszellen gehemmt wird.

5. Fusionsprotein zur Verwendung nach Anspruch 4, das sich in einer Zusammensetzung befindet, die ferner als aktiven Bestandteil einen monoklonalen Anti-SHPS-1-Antikörper enthält, der SHPS-1 spezifisch erkennt und fähig ist, daran zu binden.

## Revendications

1. Procédé *in vitro* ou *ex vivo* pour empêcher le mouvement de cellules en commandant une fonction de réponse cellulaire médiée par la SHPS-1, qui comprend la mise en contact de SHPS-1 sur la cellule avec une protéine de fusion d'un peptide partiel de CD47 et d'un fragment d'immunoglobuline Fc, laquelle protéine de fusion a une séquence d'acides aminés de SEQ ID N° 4.

2. Procédé selon la revendication 1, qui comprend en outre l'addition d'un anticorps monoclonal anti-SHPS-1 qui reconnaît spécifiquement la SHPS-1 et est capable de se lier à celle-ci.

3. Procédé selon la revendication 1 ou 2, dans lequel le procédé est utilisé pour inhiber le mouvement de cellules cancéreuses.

4. Protéine de fusion d'un peptide partiel de CD47 et d'un fragment d'immunoglobuline Fc qui a une séquence d'acides aminés de SEQ ID N° 4 pour utilisation dans un procédé pour le traitement de cancers exprimant la SHPS-1, dans laquelle le traitement est effectué par inhibition de la métastase de cellules cancéreuses.

5. Protéine de fusion pour utilisation selon la revendication 4, qui se trouve dans une composition qui contient en outre, comme ingrédient actif, un anticorps monoclonal anti-SHPS-1 qui reconnaît spécifiquement la SHPS-1 et est capable de se lier à celle-ci.
